# EUROPEAN PATENT APPLICATION

(11) **EP 4 458 879 A1**
(43) Date of publication of application: **06.11.2024**
(21) Application number: 23745804.7
(22) Date of filing: 04.01.2023
(51) Int. Cl.: C08G 63/08, C08G 63/78, C08G 64/18

(54) **POLYLACTIC ACID GRAFT COPOLYMER, AND PREPARATION METHOD THEREFOR AND USE THEREOF**

(30) Priority: 29.01.2022 CN 202210110898
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: ZHANG, Lei, Dalian, Liaoning 116045 (CN); QIAO, Kai, Dalian, Liaoning 116045 (CN); BAI, Fudong, Dalian, Liaoning 116045 (CN); SUN, Qimei, Dalian, Liaoning 116045 (CN); BAI, Yuli, Dalian, Liaoning 116045 (CN); LI, Lanpeng, Dalian, Liaoning 116045 (CN)
(74) Representative: karo IP
(86) International application number: PCT/CN2023/070327
(87) International publication number: WO 2023/142919

(57) **Abstract**

The present invention belongs to the technical field of polymer synthesis, and particularly relates to a polylactic acid graft copolymer, and a preparation method therefor and the use thereof. The polylactic acid graft copolymer is characterized in that the polylactic acid graft copolymer contains a repeating structural unit of a structure as shown in formula (A), wherein R¹ is H or a grafting group, and at least part of the R¹ in the repeating structural unit is a grafting group. In addition, the right-angle tearing strength of the polylactic acid graft copolymer is not less than 120 kN/m, and preferably 135-150 kN/m; and the elongation at break is not less than 30%, and preferably 35-45%. The grafting efficiency of the polylactic acid graft copolymer prepared by adopting the method disclosed in the technical solution of the present invention is greatly improved; the toughness, elongation at break and glass transition temperature of a polylactic acid material are improved; and a prepared grafting-modified polylactic acid copolymerization material can be green and fully biodegradable.

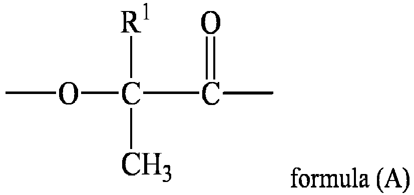

## Description

### CROSS-REFERENCE TO RELATED APPLICATIONS

The application claims the benefit of Chinese patent application No. "202210110898.2", filed on January 29, 2022, the content of which is specifically and entirely incorporated herein by reference.

### TECHNICAL FIELD

The present invention belongs to the technical field of polymer synthesis, and particularly relates to a polylactic acid graft copolymer, and a preparation method therefor, and the use thereof.

### BACKGROUND ART

The research of bio-based composite materials has been a hot topic in academia and industrial production in recent years to reduce the hazards of fossil energy sources to the natural environment. Compared with polyolefins and polyethylene terephthalate which are commonly used industrialized products, polylactic acid (PLA) has desirable strength and processability, and it is a thermoplastic resin. PLA is generated through the polymerization of lactic acid monomers, and lactic acid can be produced by the fermentation of corn, potato, cotton, hemp, and other plants. The raw materials of lactic acid are regenerative, and PLA obtained by polymerization of regenerative lactic acid has characteristics such as high strength, desirable processability, and excellent mechanical properties. However, PLA has some defects, for example, high brittleness, strong hydrophobicity, low impact strength, high price, and poor stability under high temperatures and certain humidity, the defects restrict the PLA cannot be directly used to produce the industrial products like general plastics (e.g., polyethylene, polypropylene), thus the PLA must be modified and processed. The modification processing method currently used in a widespread manner to optimize the operational performance of polylactic acid is performed by means of blending polylactic acid with other plastics, although the method is simple, it does not fundamentally alter the properties of polylactic acid material. In addition, the researchers have attempted to modify polylactic acid material per se by using other methods, such as graft modification. Since the maleic anhydride is a polar monomer material and has a five-membered cyclic structure, the maleic anhydride is a desirable graft modifier with excellent properties, and use of the maleic anhydride to perform the graft modification of polylactic acid, can on one hand improve the polarity of polylactic acid material, on the one hand, it may modify the toughness of polylactic acid macromolecular chains due to the cyclic structure of the maleic anhydride.

Currently, the method generally used in China and foreign countries for modifying properties of other materials with the maleic anhydride is as follows: firstly, using the maleic anhydride for grafting polyethylene, polystyrene, starch, lignin, and the like to obtain a pre-graft material; then adding the pre-graft material into the polylactic acid material by blending, thereby improving the toughness of polylactic acid, but the effects of such a modification method are not obvious. Some researchers have modified the nonpolar structure of polylactic acid by grafting the maleic anhydride directly to the polylactic acid macromolecular chain by means of reactive extrusion.

CN108192033A discloses a preparation method for directly grafting L-lactide/glycolide/ε-caprolactone terpolymer by maleic anhydride, the method comprises the following steps: mixing L-lactide, glycolide, ε-caprolactone according to a certain ratio and performing a polymerization reaction in the presence of a catalyst to obtain a coarse product PLLGC; dissolving the coarse product PLLGC in trichloromethane, and re-precipitating with methanol to filter out white solid, i.e., to obtain fine PLLGC. Maleic anhydride is grafted to the PLLGC copolymer by adding maleic anhydride into the fine PLLGC and carrying out a reaction in solution. Although the polylactic acid synthesized with the method has a high purity, the grafting degree of maleic anhydride is not more than 1%, and the reaction is performed through solution polymerization, which has disadvantages such as high toxicity of organic solvent in use, low molecular weight of the synthesized polymer, which is not conducive to the industrial production.

CN103570884A discloses a preparation method for maleic anhydride-modified polylactic-co-glycolide, the preparation method comprises the following steps: adding L/D-lactide, glycolide, maleic anhydride into an ampoule bottle according to a certain ratio, polymerizing the materials at the temperature of 130-170°C for 12-60h under the effect of stannous octoate catalyst, to obtain a crude product maleic anhydride-modified polylactic-co-glycolic acid (MPLGA), dissolving the MPLGA with trichloromethane, and re-precipitating in methanol, and filtering out the white solid, i.e., to obtain a fine product MPLGA. Although the polymer produced with the preparation method has a narrow molecular weight distribution, the maleic anhydride grafting reaction is performed simultaneously during the polymerization process, which causes problems such as low product grafting degree and long polymerization reaction time, the low molecular weight of the polymer, thus the method does not facilitate the industrial production.

To sum up, the maleic anhydride grafted polylactic acid in the prior art has generally suffered from the problems of low molecular weight and low grafting degree of maleic anhydride, resulting in poor mechanical properties and heat resistance.

### SUMMARY OF THE INVENTION

The present invention aims to provide a polylactic acid graft copolymer with the significantly increased molecular weight and content of the grafting group, such that the polylactic acid graft copolymer material has desirable mechanical properties, heat resistance, and green degradability, and can be easily applied in the industrial production.

In order to fulfill the above purpose, the first aspect of the present invention provides a polylactic acid graft copolymer, is characterized in that the polylactic acid graft copolymer comprises a repeating structural unit of a structure as shown in formula (A); 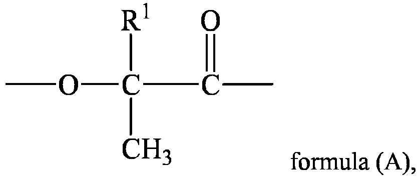 wherein R¹ is H or a grafting group, and at least part of the R¹ in the repeating structural unit is a grafting group, and the right-angle tearing strength of the polylactic acid graft copolymer is not less than 120 kN/m, preferably within the range of 135-150 kN/m; and the elongation at break is not less than 30%, preferably within the range of 35-45%.

The second aspect of the present invention provides a preparation method for a polylactic acid graft copolymer, is characterized in that the method comprises the following steps:
(1) Contacting the lactide with a grafting monomer under the conditions of nucleophilic addition reaction to obtain a nucleophilic addition product;
(2) Blending the nucleophilic addition product obtained in step (1) with a catalyst and an initiator under the conditions of ring-opening polymerization reaction to perform a low-temperature pre-polymerization to obtain a ring-opened polymerization product;
(3) Subjecting the ring-opened polymerization product obtained in step (2) to a high-temperature polymerization, in the presence of an antioxidant, under the conditions of a chain propagation reaction.

The third aspect of the present invention provides a polylactic acid graft copolymer produced with the aforementioned preparation method.

The fourth aspect of the present invention provides a use of the polylactic acid graft copolymer according to the first aspect or the third aspect in the production of agricultural films, food packaging materials, and medical hygiene products.

Compared with the prior art, the present invention provides a completely new route for the synthesis of polylactic acid graft copolymer. Firstly, lactide is graft-modified with the grafting monomers under the conditions of nucleophilic addition reaction to obtain a nucleophilic addition product including an abundant amount of grafting groups. Then, the nucleophilic addition product including an abundant amount of grafting groups is subjected to a low-temperature pre-polymerization under the conditions of ring-opening polymerization reaction to obtain a ring-opened polymerization product. Finally, the ring-opened polymerization product is subjected to a high-temperature polymerization under the conditions of a chain propagation reaction to obtain a maleic anhydride grafted polylactic acid copolymer with a high molecular weight. Compared with the method of initially performing the ring-opening copolymerization and then subjecting to the grafting in the prior art, the present invention adopts a method of initially performing grafting and then subjecting to the ring-opening copolymerization, which can effectively improve the content (i.e., grafting degree) of the grafting group, and can effectively increase the molecular weight of the polymer through the subsequent high-temperature polymerization.

Use of the preparation method for the polylactic acid graft copolymer provided in the present invention, it is theoretically possible to obtain a polylactic acid graft copolymer with a grafting degree of 50% or even 100%, and effectively regulate and control the content of grafting group in the polylactic acid graft copolymer by adjusting the ratio of raw materials, thereby regulating and controlling the properties of the polylactic acid graft copolymer.

The polylactic acid graft copolymer provided by the present invention has a high molecular weight and strong flexibility. The polylactic acid graft copolymer has an excellent right-angle tearing strength, and elongation at break, and the prepared grafting-modified polylactic acid copolymerization material can be green and fully biodegradable. Given that the terminal hydroxyl group of the polylactic acid graft copolymer is retained, thereby allowing the polylactic acid graft copolymer to be further modified through the functional group to further regulate and control the properties of the polylactic acid graft copolymer.

### BRIEF DESCRIPTION OF THE DRAWINGS

FIG. 1 shows an infrared absorption spectrum of the corresponding materials in Example 1, wherein: (a) L-lactide (LA), (b) maleic anhydride (MAH), (c) maleic anhydride grafted lactide;
FIG. 2 shows an infrared absorption spectrum of the corresponding materials in Example 1, wherein (a) polylactic acid (PLA), (b) maleic anhydride (MAH), (c) maleic anhydride grafted polylactic acid, and (d) an infrared difference spectrum for the polylactic acid subtracted from the maleic anhydride grafted polylactic acid spectrogram;
FIG. 3 shows a ¹³CNMR spectrogram of the corresponding materials in Example 1, wherein (a) L-lactide, (b) maleic anhydride grafted lactide;
FIG. 4 shows a ¹³CNMR spectrogram of the corresponding materials in Example 1, wherein (a) polylactic acid (PLA), and (b) maleic anhydride grafted polylactic acid.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

In order to achieve the above object, the first aspect of the present invention provides a polylactic acid graft copolymer, is characterized in that the polylactic acid graft copolymer comprises a repeating structural unit of a structure as shown in formula (A); 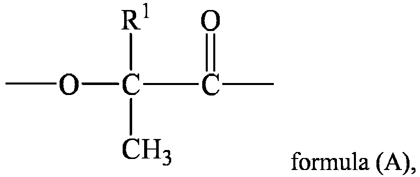 wherein R¹ is H or a grafting group, and at least part of the R¹ in the repeating structural unit is a grafting group, and the right-angle tearing strength of the polylactic acid graft copolymer is not less than 120 kN/m, preferably within the range of 135-150 kN/m; and the elongation at break is not less than 30%, preferably within the range of 35-45%.

The polylactic acid graft copolymer may also be represented by the following formula:

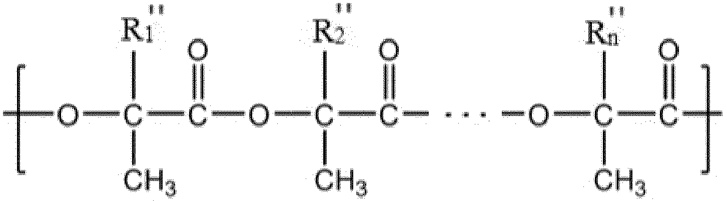

Wherein each of R₁", R₂", ...... and Rₙ" is independently selected from the group consisting of H, a grafting group represented by formula (I), and a grafting group represented by formula (II).

According to the present invention, the grafting group is contained in an amount of more than 10 mol%, preferably more than 30 mol%, and further preferably more than 40 mol%, of the total amount of R¹.

According to the present invention, the grafting groups may be various groups capable of grafting onto lactide/polylactic acid, such that the right-angle tearing strength of the polylactic acid graft copolymer is not less than 120 kN/m, preferably within the range of 135-150 kN/m; and the elongation at break is not less than 30%, preferably within the range of 35-45%; preferably, the grafting group has a structure represented by formula (I) and/or formula (II): 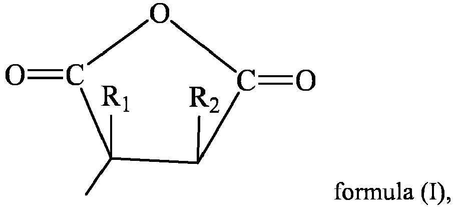 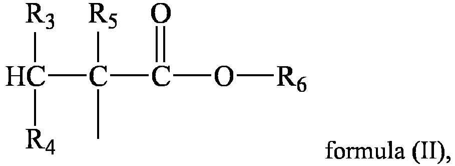 wherein each of R₁ R₂, R₃, R₄, R₅ is independently hydrogen, a substituted or unsubstituted C1-C4 alkyl group, and R₆ is a substituted or unsubstituted C1-C6 alkyl group, a C6-C20 aryl group.

In the present invention, the substituted or unsubstituted C1-C6 alkyl group may be any one selected from the group consisting of methyl, ethyl, n-propyl, isopropyl, n-butyl, sec-butyl, isobutyl, t-butyl, n-pentyl, isopentyl, t-pentyl, neopentyl, n-hexyl, and isohexyl, and the alkyl group may be substituted with one or more of a halogen, a nitro group, a primary amino group, a secondary amino group, and a tertiary amino group. The halogen may be one or more selected from the group consisting of fluorine, chlorine, bromine, and iodine.

The C6-C20 aryl group may be any one selected from the group consisting of phenyl, methylphenyl, ethylphenyl, dimethylphenyl, diphenyl, biphenyl, and polyphenyl.

According to the present invention, the maleic anhydride is taken as an example as the graft copolymer of the grafting group, ¹³CNMR of the polylactic acid graft copolymer has a peak of anhydride-based carbon atoms near the chemical shift of 169ppm and a peak of carbon atom after breaking the carbon-carbon double bond of the maleic anhydride near the chemical shift of 72 ppm, the peaks are unique for the grafting approach of the chain in the present invention. In the present invention, "near" refers to an error within the acceptable range of the art, typically ±5ppm, due to possible factors such as test equipment and personal operation. Accordingly, the method of initially performing ring-opening polymerization and then grafting in the prior art is referred to as the chain end grafting in the present invention.

According to the present invention, the polylactic acid graft copolymer has a weight average molecular weight within the range of 1×10⁵-4×10⁵. The polylactic acid graft copolymers provided by the present invention have both high molecular weights and strong flexibility. In contrast, the chain end grafted polylactic acid in the prior art has a molecular weight typically below 3×10⁵, the right-angle tearing strength below 100 kN/m, typically within the range of 80-95 kN/m, and the elongation at break no more than 10%, typically within the range of 5-10%.

According to the present invention, the glass transition temperature of the polylactic acid graft copolymer is within the range of 80-100°C. The glass transition temperature of the polylactic acid graft copolymer provided by the present invention is higher than that of the chain-end grafted polylactic acid in the prior art, which is advantageous to improve the temperature resistance of the material.

According to the present invention, at least one terminal group of the polylactic acid graft copolymer contains a hydroxyl group. As a result, the polylactic acid graft copolymer can be used for further modification by means of the functional group. The presence of the terminal hydroxyl group may be measured through a chemical titration method.

In the present invention, both the right-angle tearing strength and the elongation at break are measured by the universal mechanics experimental testing machine; the weight average molecular weight is measured through a gel chromatography method; the glass transition temperature is measured by a differential scanning calorimeter method; and the graft ratio is measured by a chemical titration method. The specific content is described in the text below.

The second aspect of the present invention provides a preparation method for a polylactic acid graft copolymer, is characterized in that the method comprises the following steps:
(1) Contacting the lactide with a grafting monomer under the conditions of nucleophilic addition reaction to obtain a nucleophilic addition product;
(2) Blending the nucleophilic addition product obtained in step (1) with a catalyst and an initiator under the conditions of ring-opening polymerization reaction to perform a low-temperature pre-polymerization to obtain a ring-opened polymerization product;
(3) Subjecting the ring-opened polymerization product obtained in step (2) to a high-temperature polymerization, in the presence of an antioxidant, under the conditions of a chain propagation reaction, in order to increase the molecular weight of the polylactic acid graft copolymer.

The present invention can effectively ensure a grafting degree by means of initially performing grafting and then performing the ring-opening polymerization and can ensure the production of a polylactic acid graft copolymer having a high molecular weight through a high-temperature polymerization, thereby providing the polymer with a high flexibility and heat resistance.

Preferably, the grafting monomer is a monomer represented by formula (i) and/or formula (ii):

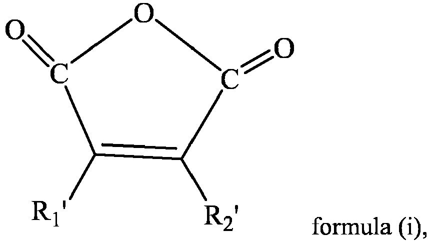

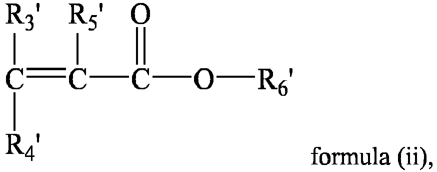

Wherein R₁', R₂', R₃', R₄', R₅', and R₆' correspond and are identical to the R_{1,} R₂, R₃, R₄, R₅, and R₆ respectively defined in the above text, the content will not be repeatedly described herein.

According to the present invention, step (1) serves to carry out a grafting reaction of lactide with a graft monomer such as maleic anhydride, whereby maleic anhydride is grafted onto lactide by cleavage of double bonds through a nucleophilic addition reaction, to obtain a grafted product. The nucleophilic addition reaction in step (1) is performed in the presence of an initiator.

The lactide described in the present invention is at least one selected from the group consisting of L-lactide, D-lactide, and meso-lactide, preferably L-lactide and/or D-lactide.

According to the present invention, the initiator is a peroxide initiator, preferably dicumyl peroxide and/or 2,5-dimethyl-2,5-bis-(t-butyl-peroxy) hexane.

According to the present invention, the initiator is added in an amount of 0.1-5wt%, preferably 0.5-2wt%, of the lactide dosage.

According to the present invention, step (1) is a constant temperature reaction carried out under an inert gas flow, preferably a nitrogen gas flow, and the vacuum condition; the conditions of the nucleophilic addition reaction comprise a reaction temperature within the range of 50-150°C, preferably within the range of 80-120°C, a reaction pressure within the range of 2-100 kPa, preferably within the range of 20-60kPa, a reaction time within the range of 0.5-10h, preferably within the range of 2-6h; the flow rate of the protective nitrogen gas flow within the range of 0.5-6m/s, preferably within the range of 2-4m/s.

Unless otherwise specified in the present invention, the pressure refers to an absolute pressure.

According to the present invention, the optical purity of the lactide in step (1) may be within the range of 99.0-99.6%, preferably within the range of 99.4-99.6%, both the self-prepared and the purchased lactide can be used herein.

According to the present invention, to obtain better toughness and increase the grafting degree of the grafting group (i.e., the content of the grafting group in the polylactic acid material), the addition amount of the grafting monomer is preferably 100-300wt%, more preferably 150-200wt% , of the lactide dosage.

Taking maleic anhydride and L-lactide as examples, the main reactions carried out in step (1) are represented in formula (a) and formula (b) as follows:

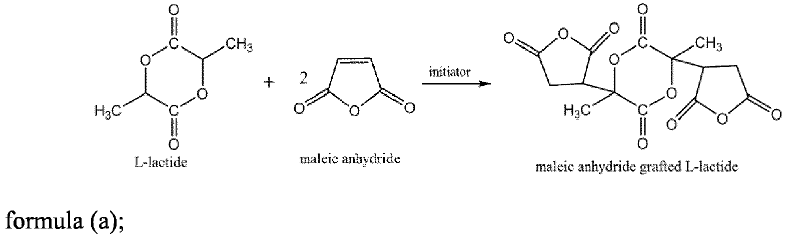

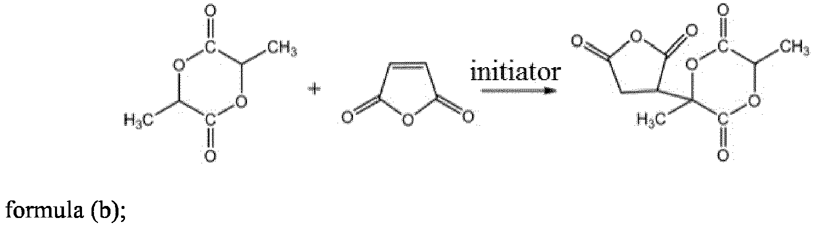

Theoretically, the grafting degree of the obtained product represented by formula (a) is 100%, and the grafting degree of the obtained product represented by formula (b) is 50%.

According to the present invention, the method further comprises: removing the lactide not participating in the nucleophilic addition reaction through the vacuum distillation process after step (1), then subjecting the obtained product to the reaction in step (2).

According to the present invention, the conditions of vacuum distillation comprise: a temperature within the range of 100-200°C, preferably within the range of 120-150°C; a pressure within the range of 0-100 kPa, preferably within the range of 10-50kPa; and a time within the range of 1-10 h, preferably within the range of 2-6h.

According to the present invention, the catalyst of step (2) is used for catalyzing a ring-opening reaction of the lactide after grafting the grafting group, preferably at least one selected from the group consisting of stannous octoate, zinc lactate, tri-alkyl aluminum, tri-isobutyl aluminum, and stannous chloride.

According to the present invention, the catalyst is added in an amount of 0.1-5wt%, preferably 0.5-3wt%, of the total weight of the nucleophilic product.

According to the present invention, the initiator of step (2) is an alkyl hydroxyl group or an aromatic hydroxyl group-type initiator for initiating the polymerization of lactide after introducing the grafting group following the ring-opening reaction, the initiator is preferably at least one selected from the group consisting of glycerol, xylitol, ethylene glycol and tri-phenyl phosphine, and tri-phenyl phosphine is preferred.

According to the present invention, the initiator is added in an amount of 0.05-5wt%, preferably 0.3-2wt%, of the total weight of the nucleophilic product.

According to the present invention, the low-temperature pre-polymerization in step (2) is performed in an inert gas flow, preferably a nitrogen gas flow, under the negative pressure condition; the conditions of the ring-opening polymerization reaction in step (2) comprise: a reaction temperature within the range of 100-150°C, preferably within the range of 120-140°C; a reaction pressure within the range of 100-1,000 kPa, preferably within the range of 200-500 kPa; a reaction time within the range of 2-15h, preferably within the range of 3-8h; and a gas flow rate of the nitrogen gas flow within the range of 0.5-6m/s, preferably within the range of 2-4m/s.

According to the present invention, the antioxidant in step (3) is used for preventing oxidation of the polylactic acid oligomer of step (2), thereby ensuring further polymerization at higher temperatures and chain propagation reaction. The antioxidant may be various substances capable of preventing oxidation of polylactic acid at high-temperature without adversely affecting the polylactic acid material, for example, the antioxidant may be at least one selected from the group consisting of phosphites antioxidant, alkylpolyphenol antioxidant, and thiobisphenol antioxidants, preferably triphenyl phosphite.

According to the present invention, the antioxidant is added in an amount of 0.1-5wt%, preferably 0.5-2wt%, of the total weight of the ring-opened polymerization product.

According to the present invention, the temperature of the chain propagation reaction in step (3) is within the range of 140-250°C, preferably within the range of 160-200°C.

According to the present invention, the step (3) is preferably performed in a twin screw extruder. The reaction process is performed by using a twin screw extruder, wherein the ring-opened polymerization product and the antioxidant are added into the twin screw extruder for carrying out the high-temperature reactive mixing, extrusion granulation, and the polylactic acid graft copolymer is finally obtained.

The high-temperature polymerization conditions comprise: an inlet temperature of the twin screw extruder is within the range of 140-160°C, and an outlet temperature of the twin screw extruder is within the range of 170-190°C. The residence time of the material in the twin screw extruder is preferably within the range of 13-23 min.

The preparation method for polylactic acid graft copolymer provided by the present invention includes a bulk pre-polymerization reaction of lactide in a reaction kettle, coupling with the copolymerization in the twin screw extruder, thus the reaction operation is simple and can be easily applied in the industrial production.

Taking maleic anhydride and L-lactide as examples, the main reaction processes of steps (2) and (3) are represented in formula (c) and formula (d) as follows:

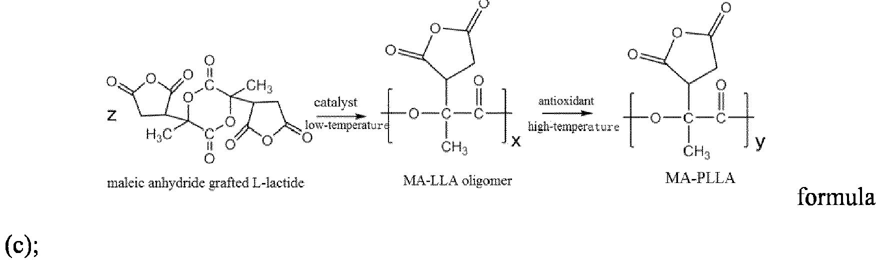

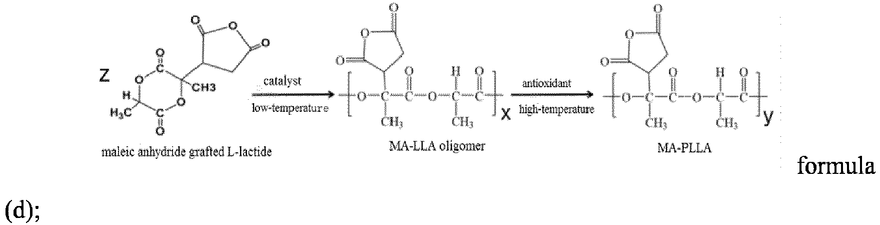

In the above formula (c) and formula (d), x and y denote the degree of polymerization of MA-LLA, y is greater than x, and z denotes the number of moles.

The third aspect of the present invention provides a polylactic acid graft copolymer produced with the aforementioned preparation method.

The polylactic acid graft copolymer produced with the aforementioned preparation method has right-angle tearing strength within the range of 120-150 KN/m, elongation at break within the range of 30-45%, the glass transition temperature within the range of 80-100°C, the molecular weight within the range of 1×10⁵-4×10⁵, and a grafting degree within the range of 40-50%.

The fourth aspect of the present invention provides a use of the polylactic acid graft copolymer according to the first aspect or the third aspect in the production of agricultural films, food packaging materials, and medical hygiene products.

The polylactic acid graft copolymer can be more favorably used in the production of agricultural films, food packaging materials, and medical hygiene products because the copolymer has a higher grafting degree, thus it can have desirable flexibility and temperature resistance while ensuring high molecular weight.

The present invention will be described in detail below with reference to examples. Unless otherwise specified in the following examples, the reagents in use are commercially available products in the industry.

The test methods relating to performance parameters in the present invention were described as follows:

### (1) Grafting degree of the grafting groups:

The grafting degree of grafting groups in polylactic acid graft copolymer was measured through the chemical titration method, in particular: a certain mass of polylactic acid graft copolymer was weighed and placed in a cone-shaped bottle, and dissolved by adding a certain amount of tetrahydrofuran, a few droplets of thymol blue/DMF indicator was dropwise added; then titrated with an excess amount ethanol solution of potassium hydroxide having a concentration of 0.05mol/L, and the back titration was performed with the HCl-isopropanol solution having a concentration of 0.01mol/L.

Grafting degree = n₂/n₁×100%, n₁= (m₀-m₂)/M₁, n₂= m₂/M₂, i.e., grafting degree = [(0.05V_{KOH}-0.01V_{HCl})×2×M₁]/[m₀-(0.05V_{KOH}-0.01V_{HCl}) ×2×M₂]; wherein m₀ denoted the mass of the weighted polylactic acid graft copolymer, in a unit of gram (g); m₂ denoted the total mass of the grafting groups on the molecular chain of polylactic acid, it was calculated according to the amount of consumed KOH; n₁ denoted the degree of polymerization of polylactic acid, i.e., the amount of substance of lactic acid units, n₂ denoted the amount of substance of the grafting groups on the molecular chain of polylactic acid, M₁ denoted the molar mass of lactic acid, M₂ denoted the molar mass of the grafting group, V_{KOH} denoted the volume of the added ethanol solution of potassium hydroxide, in a unit of mL; V_{HCl} denoted the volume of the HCl-isopropanol solution consumed by titration, in a unit of mL.

(2) Right-angle tearing strength, and elongation at break: were measured by using a universal mechanical testing machine according to the National Standard GB/T1039-1992 of China.

(3) Detection of terminal hydroxyl group: was measured with the phthalic anhydride method according to the National Standard GB/T12008.3-2009 of China.

(4) Glass transition temperature: was measured with the Differential Scanning Calorimeter (DSC) (DSC 204, manufactured by the NETZSCH of Germany). The test conditions were as follows: 5-10 mg of the sample was weighted, the sample was initially heated from room temperature to 200°C at a temperature-rise rate of 10°C /min, and isothermally preserved for 5min to eliminate the thermal history of the sample; the sample was subsequently rapidly cooled down to -20°C, and then further heated from -20°C to 200°C at a temperature-rise rate of 10°C /min; nitrogen gas was introduced as a protective gas throughout the entire testing process, and the flow rate of nitrogen gas was set to 50mL/min.

(5) Weight average molecular weight of the polymer: was measured with the Gel Permeation Chromatograph (GPC), and the molecular weight of the polylactic acid graft copolymer sample was characterized by using the Gel Permeation Chromatograph (GPC Waters 1515 system). Chromatographically pure THF was the mobile phase, the flow rate was 1.0mL/min, and the column temperature was 35°C. Before testing, the sample was dissolved in THF at a concentration of 5mg/mL, the sample injection was performed after the sample was filtered by using the Polytetrafluoroethylene (PTFE) filter membrane having a pore size of 0.22µm, the sample was calibrated with the Polystyrene (PS) standard having a narrow distribution.

### Example 1

(1) L-lactide and maleic anhydride were added into a reaction kettle, an initiator dicumyl peroxide was further added, and the nucleophilic addition was carried out at constant temperature under nitrogen gas flow and the vacuuming conditions. The nucleophilic addition reaction was performed under conditions such as the reaction temperature of 105°C, the reaction pressure of 45kPa, the reaction time of 3.5h, and the gas flow rate of 2m/s for nitrogen gas flow; the maleic anhydride was added in an amount of 155wt% of the L-lactide dosage, and the dicumyl peroxide was added in an amount of 0.7wt% of the L-lactide dosage.
(2) The L-lactide not participating in the nucleophilic addition reaction of step (1) was removed through vacuum distillation to obtain a purified nucleophilic addition product; the vacuum distillation was performed at the temperature of 130°C and pressure of 25kPa for a time of 3.5h. The purified product detected with chromatography was maleic anhydride grafted L-lactide (MA-LLA).
(3) The maleic anhydride grafted L-lactide (MA-LLA) obtained from step (2) was subjected to a ring-opening polymerization reaction under a nitrogen gas flow atmosphere and the effects of a catalyst and an initiator, and the ring-opening polymerization product (i.e., the MA-LLA oligomer) was obtained through the low-temperature pre-polymerization. The ring-opening polymerization reaction was performed under conditions such as a reaction temperature of 140°C, a reaction pressure of 350kPa, a reaction time of 4.5h, and a gas flow rate of nitrogen gas flow being 3.5m/s; the addition amount of the catalyst stannous octoate was 0.75wt% of the MA-LLA dosage; and the addition amount of the initiator tri-phenyl phosphine was 0.35wt% of the MA-LLA dosage. The weight average molecular weight of the MA-LLA oligomer was 5,500.
(4) The MA-LLA oligomer obtained in step (3) was added together with an antioxidant into a twin screw extruder, and subjected to a high-temperature reactive mixing process, such that the MA-LLA oligomer was subjected to a chain propagation reaction, extrusion granulation, and finally obtained the polylactic acid graft copolymer, i.e., maleic anhydride grafted polylactic acid copolymer material (MA-PLLA). The addition amount of the antioxidant triphenyl phosphite was 0.65wt% of the MA-LLA oligomer dosage, based on the weight of the MA-LLA oligomer, and the reaction extrusion temperature of the twin screw extruder for reactive mixing and extrusion granulation was 185°C, and the residence time of materials in the twin screw extruder was 15 min.

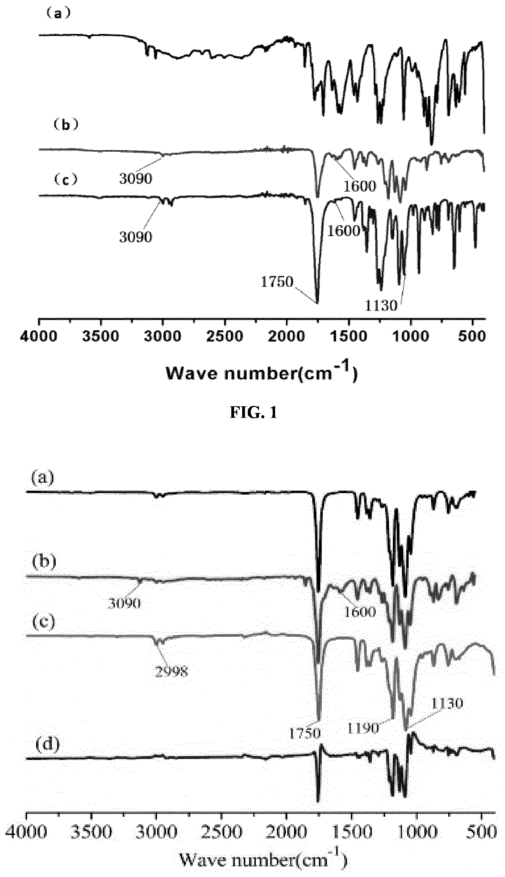

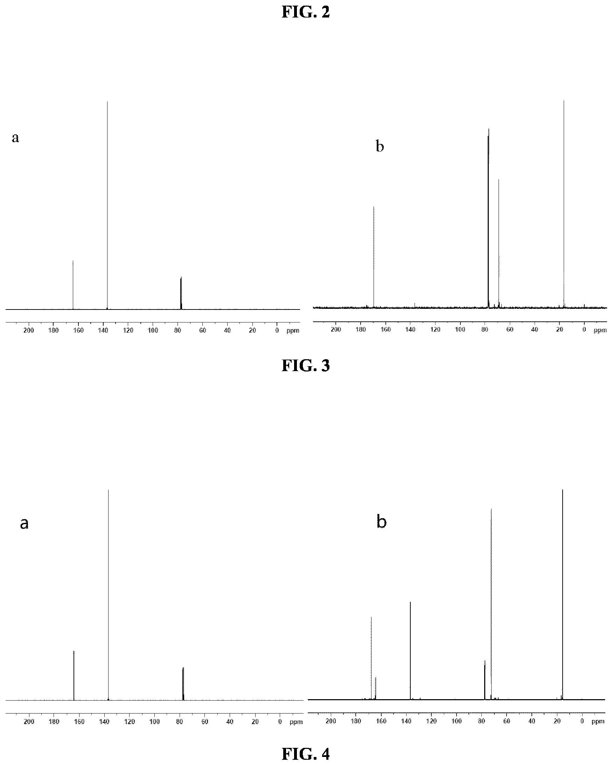

Wherein the infrared spectrograms of L-lactide, maleic anhydride, and maleic anhydride grafted lactide were shown in curves (a), (b), and (c) of FIG. 1, respectively; the infrared spectrograms of PLA, maleic anhydride, maleic anhydride grafted polylactic acid (MAH-g-PLA), and an infrared difference spectrum for the polylactic acid subtracted from the maleic anhydride grafted polylactic acid spectrogram were illustrated in curves (a), (b), (c), and (d) of FIG. 2, respectively; the ¹³CNMR spectrogram of L-lactide, and maleic anhydride grafted lactide were shown in curves (a), (b) of FIG. 3, respectively; and the ¹³CNMR spectrogram of polylactic acid and maleic anhydride grafted polylactic acid was shown in curves (a), (b) of FIG. 4, respectively.

As can be seen from FIG. 1, the infrared spectrogram of the maleic anhydride grafted lactide showed an out-of-plane bending vibration peak of an alkene-hydrogen =C-H at 3090cm⁻¹ and a stretching vibration peak of -C=C-at 1600cm⁻¹, the peaks indicated that the maleic anhydride was successfully grafted onto the lactide.

As can be seen from FIG. 2, the infrared difference spectrum between the MAH-g-PLA and pure PLA indicated an absorption peak of C=O group at 1750 cm⁻¹; and the absorption peaks of C-O-C groups at 1190cm⁻¹, 1130cm⁻¹ and 1093cm⁻¹, respectively; the absorption peaks of the carbonyl and ether linkages are enhanced in the infrared spectrogram of MAH-g-PLA compared to PLA, the peaks indicated that MAH was successfully grafted onto the PLA macromolecular chain.

As illustrated in curves (a) and (b) of FIG. 3, the peaks of two kinds of carbon atoms in the anhydride group were increased at the chemical shifts 70ppm and 18ppm, respectively, the peaks indicated that the maleic anhydride was successfully grafted onto L-lactide.

As can be seen from the comparison of curve (a) and curve (b) of FIG. 4, there was a peak of anhydride-based carbon atom at the chemical shift of 169ppm, and a peak of carbon atom after the cleavage of carbon-carbon double bond of the maleic anhydride at the chemical shift of 72ppm, the peaks indicated that MAH was successfully grafted onto the PLA macromolecular chain.

The presence of terminal hydroxyl groups was determined through the chemical titration method.

### Examples 2-11

The polylactic acid graft copolymer was prepared according to similar steps and methods as those in Example 1, except for the reaction conditions, type of materials, and addition amounts of materials, the specific details were shown in Table 1.

### Example 12

The polylactic acid graft copolymer was prepared according to the same method as that in Example 1, except that the nucleophilic addition reaction conditions comprised: the temperature of 170°C and the reaction pressure of 5kPa.

### Example 13

The polylactic acid graft copolymer was prepared according to the same method as that in Example 1, except that the addition amount of the maleic anhydride was 310wt% of the L-lactide dosage, and the addition amount of dicumyl peroxide was 6wt% of the L-lactide dosage.

### Example 14

The polylactic acid graft copolymer was prepared according to the same method as that in Example 1, except that the addition amount of the catalyst stannous octoate in the ring-opening polymerization reaction was 5wt% of the dosage of nucleophilic addition product, and the addition amount of the initiator tri-phenyl phosphine was 5wt% of the dosage of nucleophilic addition product.

### Example 15

The polylactic acid graft copolymer was prepared according to the same method as that in Example 1, except that the addition amount of an antioxidant in the chain propagation reaction was 5wt% of the dosage of the ring-opened polymerization product, and the ring-opening polymerization reaction temperature was 250°C.

### Example 16

The polylactic acid graft copolymer was prepared according to the same method as that in Example 1, except that maleic anhydride was replaced with methyl methacrylate.

### Example 17

The polylactic acid graft copolymer was prepared according to the same method as that in Example 1, except that the L-lactide in the nucleophilic addition reaction was replaced with D-lactide.

### Comparative Example 1

The polylactic acid graft copolymer was prepared with the method of initially polymerizing L-lactide and then carrying out the grafting process in the prior art:
(1) L-lactide (LLA) was subjected to a low-temperature pre-polymerization under the effects of a catalyst and an initiator and under nitrogen gas flow and negative pressure conditions to obtain a LLA oligomer. The low-temperature pre-polymerization was performed with a reaction temperature of 130°C, a reaction time of 3h, a reaction pressure of 300kPa, and a flow rate of nitrogen gas flow being 3m/s; the addition amount of the catalyst stannous octoate was 0.5wt% of the LLA dosage; and the addition amount of the initiator tri-phenyl phosphine was 0.2wt% of the LLA dosage, of the weight of L-lactide (LLA).
(2) The LLA oligomer obtained in step (1) was added together with the Maleic Anhydride (MA) and an antioxidant into a twin screw extruder for carrying out a polymerization reaction, and subjected to a high-temperature reactive mixing and extrusion granulation process so that a maleic anhydride grafted polylactic acid material (MA-PLLA) was finally obtained; the addition amount of the maleic anhydride was 2.5wt% of the dosage of LLA oligomer, the addition amount of the antioxidant triphenyl phosphite was 0.5wt% of the dosage of the LLA oligomer, of the weight of the LLA oligomer, and the reaction extrusion temperature of the twin screw extruder for reactive mixing and extrusion granulation was 180°C.

The presence of terminal hydroxyl groups was not detected through the chemical titration method.

### Comparative Example 2

The polymer was prepared by using the method disclosed in Example 1 of CN104371082A.

30g of lactide, 3g of maleic anhydride, 9mg of stannous octoate, 3mg of benzoyl peroxide, and 50ml of toluene were added into an autoclave, nitrogen gas was introduced into the autoclave for 5 min to remove oxygen gas, the reaction was carried out under the air-tight condition at 200°C for 20h, then cooled with water, the autoclave was opened, the solution was poured into a part of anhydrous methanol, the unreacted substances were removed by stirring, then subjected to the suction filtration, the powder obtained from the suction filtration was subjected to the vacuum drying to prepare a polylactic acid maleic anhydride modified substance, which was un-discolored.

The presence of terminal hydroxyl groups was determined through the chemical titration method.

### Comparative Example 3

The polymer was prepared using the method disclosed in Example 1 of CN104371082A, except that the addition amount of maleic anhydride was 140wt% of the L-lactide dosage.

The presence of terminal hydroxyl groups was determined through the chemical titration method.

**Table 1**

| | | Example 1 | Exampl e 2 | Exampl e 3 | Exampl e 4 | Exampl e 5 | Example 6 |
|---|---|---|---|---|---|---|---|
| Nucleophil ic addition reaction | | L-lactide | | | | | |
| | Grafting groups | Maleic anhydride | | | | | 2-methyl maleic anhydrid e |
| | Initiator | Dicumyl peroxide | | | | | |
| | Reaction temperature (°C) | 105 | 120 | 85 | 100 | 110 | 105 |
| | Reaction pressure (kPa) | 45 | 60 | 25 | 40 | 55 | 45 |
| | Reaction time (h) | 3.5 | 2 | 6 | 3 | 4 | 3.5 |
| | Grafting group/lactide (wt%) | 155 | 180 | 200 | 150 | 160 | 155 |
| | Initiator/lacti de (wt%) | 0.7 | 2 | 1.5 | 0.65 | 0.8 | 0.7 |
| vacuum distillation | Temperature (°C) | 130 | 145 | 125 | 130 | 135 | 130 |
| | Pressure (kPa) | 25 | 40 | 50 | 20 | 30 | 25 |
| | Time (h) | 3.5 | 6 | 5 | 3 | 4 | 3.5 |
| Ring-opene d polymeriza tion reaction | Catalyst | Stannous octoate | | | | | |
| | Initiator | Tri-phenyl phosphine | | | | | |
| | Reaction temperature (°C) | 140 | 120 | 130 | 135 | 140 | 140 |
| | Reaction time (h) | 4.5 | 8 | 6 | 6 | 5 | 4.5 |
| | Reaction pressure (kPa) | 350 | 500 | 230 | 350 | 400 | 350 |
| | Catalyst / nucleophilic addition product (wt%) | 0.75 | 3 | 1.5 | 0.65 | 0.85 | 0.75 |
| | Initiator / nucleophilic addition product (wt%) | 0.35 | 1.4 | 2 | 0.3 | 0.4 | 0.35 |
| Chain propagatio n reaction | Antioxidant | Triphenyl phosphite | | | | | |
| | Antioxidant / ring-opened polymerizati on product (wt%) | 0.65 | 1 | 2 | 0.7 | 0.7 | 0.65 |
| | Temperature (°C) | 185 | 160 | 165 | 185 | 190 | 185 |
| | Time (min) | 15 | 16 | 17 | 17 | 18 | 17 |

**Table 1 (continued)**

| | | Example 7 | Example 8 | Example 9 | Example 10 | Example 11 |
|---|---|---|---|---|---|---|
| Nucleophi lie addition reaction | | L-lactide | | | | |
| | Grafting groups | Maleic anhydride | | | | |
| | Initiator | 2,5-dimeth yl-2,5-bis-(t-butylperox y) hexane | Dicumyl peroxide | Dicumyl peroxide | 2,5-dime thyl-2,5-bis-(t -butylper oxy) hexane | Dicumyl peroxide |
| | Reaction temperature (°C) | 110 | 95 | 100 | 95 | 100 |
| | Reaction pressure (kPa) | 55 | 35 | 35 | 35 | 40 |
| | Reaction time (h) | 4 | 2 | 3 | 2 | 3 |
| | Grafting group/ lactide (wt%) | 160 | 140 | 145 | 140 | 150 |
| | Initiator/ lactide (wt%) | 0.8 | 0.5 | 0.6 | 0.5 | 0.65 |
| vacuum distillatio n | Temperatur e(°C) | 135 | 125 | 130 | 125 | 130 |
| | Pressure (kPa) | 30 | 15 | 15 | 15 | 20 |
| | Time (h) | 3.5 | 2 | 2.5 | 2 | 3 |
| Ring-open ed polymeriz ation reaction | Catalyst | Stannous octoate | Stannous octoate | Stannous octoate | Zinc lactate | Tri-isobut yl aluminu m |
| | Initiator | Glycerol | Tri-phen yl phosphin e | Tri-phen yl phosphin e | Glycerol | Ethylene glycol |
| | Reaction temperature (°C) | 145 | 130 | 135 | 130 | 135 |
| | Reaction time (h) | 5 | 3 | 4 | 3 | 4.5 |
| | Reaction pressure (kPa) | 400 | 300 | 350 | 300 | 350 |
| | Catalyst / nucleophilic addition product (wt%) | 0.8 | 0.5 | 0.6 | 0.5 | 0.65 |
| | Initiator / nucleophilic addition product (wt%) | 0.8 | 0.2 | 0.25 | 0.2 | 0.3 |
| Chain propagati on reaction | Antioxidant | Thio bisphenol | Tripheny l phosphit e | Tripheny l phosphit e | Thio bispheno l | Triphenyl phosphite |
| | Antioxidant / ring-opened polymerizat ion product (wt%) | 0.7 | 0.5 | 0.6 | 0.5 | 0.4 |
| | Temperatur e (°C) | 190 | 180 | 180 | 180 | 185 |
| | Time (min) | 18 | 20 | 19 | 18 | 17 |

**Table 2**

| | Right-angle tearing strength (KN/m) | Elongation at break (%) | Glass transition tempera ture (°C) | Weight average molecular weight of ring-opened polymerizati on product | Weight average molecular weight of the product (×10⁵) | Grafting degree (%) |
|---|---|---|---|---|---|---|
| Example 1 | 145 | 40 | 96 | 5500 | 3.05 | 49.7 |
| Example 2 | 143 | 39 | 95 | 5600 | 2.98 | 49.1 |
| Example 3 | 144 | 39 | 95 | 5200 | 2.85 | 49.5 |
| Example 4 | 144 | 39 | 94 | 5400 | 2.9 | 49.2 |
| Example 5 | 145 | 38 | 93 | 5300 | 2.82 | 49.3 |
| Example 6 | 145 | 39 | 95 | 5500 | 3.02 | 49.5 |
| Example 7 | 132 | 35.7 | 90.5 | 5300 | 2.89 | 40.2 |
| Example 8 | 130 | 35 | 90 | 5200 | 2.5 | 42.5 |
| Example 9 | 135 | 37 | 92 | 5100 | 2.7 | 44.6 |
| Example 10 | 125 | 30 | 88 | 5400 | 2.13 | 40.2 |
| Example 11 | 132 | 34 | 90.5 | 5300 | 2.74 | 45.6 |
| Example 12 | 128 | 33 | 88 | 5100 | 2.34 | 41.4 |
| Example 13 | 129 | 33 | 89 | 5200 | 2.42 | 41.8 |
| Example 14 | 133 | 36 | 91 | 5200 | 2.62 | 43.5 |
| Example 15 | 134 | 36 | 91 | 5200 | 2.65 | 43.8 |
| Example 16 | 131 | 36 | 90 | 5300 | 2.52 | 43 |
| Example 17 | 144 | 40 | 95 | 5400 | 3.01 | 49.2 |
| Comparat ive Example 1 | 95 | 8 | 68 | - | 2.01 | 2 |
| Comparat ive Example 2 | 103.2 | 12.5 | 104.2 | - | 2.61 | 13.8 |
| Comparat ive Example 3 | 110 | 18 | 99 | - | 2.25 | 30.4 |

It is illustrated from Table 2 that use of the method disclosed in the present invention, the grafting reaction of grafting groups is performed before the polymerization of lactide, so that the grafting efficiency is greatly improved, the toughness, the elongation at break, and the glass transition temperature of the polylactic acid material are enhanced, and the prepared grafting-modified polylactic acid copolymerization material can be green and fully biodegradable.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A polylactic acid graft copolymer, is **characterized in that** the polylactic acid graft copolymer comprises a repeating structural unit of a structure as shown in formula (A); 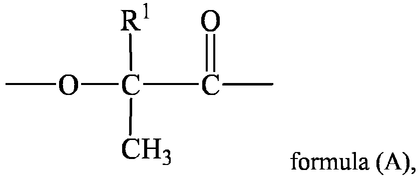 wherein R¹ is H or a grafting group, and at least part of the R¹ in the repeating structural unit is a grafting group, and the right-angle tearing strength of the polylactic acid graft copolymer is not less than 120 kN/m, preferably within the range of 135-150 kN/m; and the elongation at break is not less than 30%, preferably within the range of 35-45%.

2. The polylactic acid graft copolymer according to claim 1, wherein the grafting group is contained in an amount of more than 10 mol%, preferably more than 30 mol%, and further preferably more than 40 mol%, of the total amount of R¹.

3. The polylactic acid graft copolymer according to claim 1 or 2, wherein the grafting group has a structure represented by formula (I) and/or formula (II): 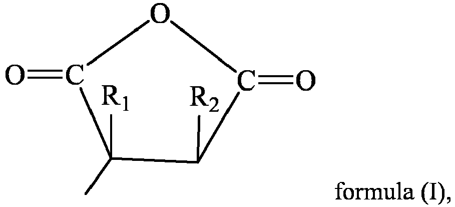 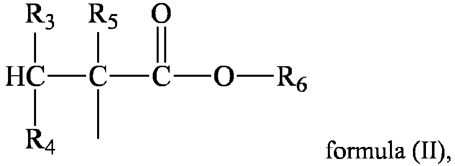 wherein each of R₁, R₂, R₃, R₄, R₅ is independently hydrogen, a substituted or unsubstituted C1-C4 alkyl group, and R₆ is a substituted or unsubstituted C1-C6 alkyl group, a C6-C20 aryl group.

4. The polylactic acid graft copolymer according to any of claims 1-3, wherein ¹³CNMR of the polylactic acid graft copolymer has a peak near the chemical shift of 169ppm and a peak near the chemical shift of 72ppm.

5. The polylactic acid graft copolymer according to any of claims 1-4, wherein the polylactic acid graft copolymer has a weight average molecular weight within the range of 1×10⁵-4×10⁵.

6. The polylactic acid graft copolymer according to any of claims 1-5, wherein the glass transition temperature of the polylactic acid graft copolymer is within the range of 80-100°C.

7. The polylactic acid graft copolymer according to any of claims 1-6, wherein the polylactic acid graft copolymer contains a terminal hydroxyl group measured by using the phthalic anhydride method.

8. A preparation method for a polylactic acid graft copolymer, is **characterized in that** the method comprises the following steps:
(1) Contacting the lactide with a monomer represented by formula (i) and/or formula (ii) under the conditions of nucleophilic addition reaction to obtain a nucleophilic addition product;
(2) Blending the nucleophilic addition product obtained in step (1) with a catalyst and an initiator under the conditions of ring-opening polymerization reaction to perform a low-temperature pre-polymerization to obtain a ring-opened polymerization product;
(3) Subjecting the ring-opened polymerization product obtained in step (2) to a high-temperature polymerization, in the presence of an antioxidant, under the conditions of a chain propagation reaction; 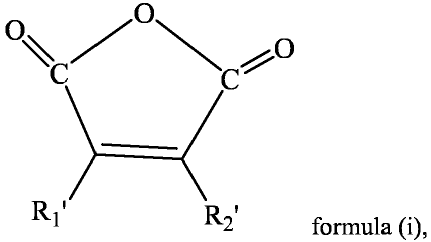 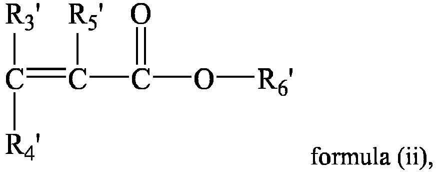 wherein each of R₁', R₂', R₃', R₄', R₅' is independently hydrogen, a substituted or unsubstituted C1-C4 alkyl group, and R₆' is a substituted or unsubstituted C1-C6 alkyl group, a C6-C20 aryl group.

9. The preparation method according to claim 8, wherein the nucleophilic addition reaction in step (1) is performed in the presence of an initiator;
preferably, the initiator is dicumyl peroxide and/or 2,5-dimethyl-2,5-bis-(t-butyl-peroxy) hexane.

10. The preparation method according to claim 9, wherein the initiator is added in an amount of 0.1-5wt%, preferably 0.5-2wt%, of the lactide dosage.

11. The preparation method according to any one of claims 8-10, wherein the conditions of the nucleophilic addition reaction comprise: a reaction temperature within the range of 50-150°C, a reaction pressure within the range of 2-100 kPa, and a reaction time within the range of 0.5-10 hours.

12. The preparation method according to any one of claims 8-11, wherein the sum of addition amounts of the monomers represented by formula (i) and formula (ii) is 100-300wt%, preferably 150-200wt%, of the lactide dosage.

13. The preparation method according to any one of claims 8-12, wherein the method further comprises: removing the lactide not participating in the nucleophilic addition reaction through vacuum distillation process after step (1), then subjecting the obtained product to the reaction in step (2);
preferably, the conditions of vacuum distillation comprise: a temperature within the range of 100-200°C, a pressure within the range of 0-100 kPa, and a time within the range of 1-10 hours.

14. The preparation method according to any one of claims 8-13, wherein the catalyst in step (2) is at least one selected from the group consisting of stannous octoate, zinc lactate, tri-alkyl aluminum, tri-isobutyl aluminum, and stannous chloride;
preferably, the catalyst is added in an amount of 0.1-5wt%, preferably 0.5-2wt%, of the total weight of the nucleophilic product;
and/or, the initiator is at least one selected from the group consisting of glycerol, xylitol, ethylene glycol, and tri-phenyl phosphine;
preferably, the initiator is added in an amount of 0.05-5wt%, preferably 0.3-2wt%, of the total weight of the nucleophilic product.

15. The preparation method according to any one of claims 8-14, wherein the conditions of the ring-opening polymerization reaction in step (2) comprise: a reaction temperature within the range of 100-150°C, a reaction pressure within the range of 100-1,000 kPa, and a reaction time within the range of 2-15h.

16. The preparation method according to any one of claims 8-15, wherein the antioxidant in step (3) is at least one selected from the group consisting of phosphites, alkylpolyphenol, and thiobisphenol antioxidants;
preferably, the antioxidant is added in an amount of 0.1-5wt%, preferably 0.5-2wt%, of the total weight of the ring-opened polymerization product.

17. The preparation method according to any one of claims 8-16, wherein a temperature of the chain propagation reaction in step (3) is within the range of 140-250°C, preferably within the range of 160-200°C.

18. The preparation method according to any one of claims 8-17, wherein step (3) is performed in a twin screw extruder.

19. The polylactic acid graft copolymer produced with the preparation method according to any one of claims 8-18.

20. Use of the polylactic acid graft copolymer according to any one of claims 1-7 and 19 in the production of agricultural films, food packaging materials, and medical hygiene products.
